# EUROPEAN PATENT APPLICATION

(11) **EP 4 252 882 A1**
(43) Date of publication of application: **04.10.2023**
(21) Application number: 22166113.5
(22) Date of filing: 31.03.2022
(51) Int. Cl.: B01D 15/18, B01D 15/36, B01D 61/04, B01D 61/14

(54) **SEPARATION SYSTEM FOR SEPARATING AND PURIFYING A TARGET COMPONENT**

(71) Applicant: Sartorius Stedim Biotech GmbH, 37079 Göttingen (DE)
(72) Inventor: Kruse, Thomas, 37308 Heilbad Heiligenstadt (DE); Schmitz, Fabian, 34121 Kassel (DE); Kampmann, Markus, 44149 Dortmund (DE)
(74) Representative: Müller-Boré & Partner Patentanwälte PartG mbB

(57) **Abstract**

The present invention relates to a separation system for separating and purifying a target component, a method for separating and purifying a target component, and the use of a single-pass crossflow diafiltration unit for integrally connecting a first and a second chromatography device.

## Description

The present invention relates to a separation system for separating and purifying a target component, a method for separating and purifying a target component, and the use of a single-pass crossflow diafiltration unit for integrally connecting a first and a second chromatography device.

In recent years, there have been achieved large improvements in the upstream process of target component producing cell lines. This led to the situation that the bottleneck in production was shifted to the subsequent clarification, capture and purification unit operations in the downstream process (DSP).

In the downstream process of biopharmaceutical products, purification is commonly performed by chromatography unit operations in bind and elute mode. In this case, the product binds to the ligand of the chromatography medium while impurities like aggregates, host cell proteins (HCP) and desoxyribonucleic acid (DNA) do not bind and therefore flow through the chromatography device. Afterwards, the product is recovered by an elution step with specific buffer conditions and is thereby separated from the impurities.

However, this process is time-consuming and cost-intensive because different steps like loading, washing, elution, cleaning and equilibration have to be performed using several different buffer systems. Further disadvantages of this operation mode are increased footprints for different chromatography systems, a high complexity of the operation, and the challenge of integration into continuous production processes.

A flow-through operation mode offers several advantages. By selecting appropriate buffer conditions, the product flows through the chromatography medium while the impurities are retained. This reduces the complexity of the operation and allows an easy integration into a continuous process. However, between different flow-through chromatography unit operations, the buffer conditions need to be changed to allow an efficient separation of the impurities from the product. This is commonly achieved by an additional unit operation such as diafiltration/tangential flow filtration (TFF) which in turn leads to disadvantages, as it makes the overall process more complex, time consuming and expensive.

Thus, the technical problem underlying the present invention is to provide means for downstream processing of target components, whereby said means should be rather inexpensive, should have low complexity and should still provide high purities and yields when compared to conventional means for DSP.

The solution to the above technical problem is achieved by the embodiments characterized in the claims.

In particular, the present invention relates to a separation system configured to process a first fluid containing a plurality of components, wherein at least one component of the plurality of components of the first fluid is a target component and wherein the separation system comprises:
a first and a second chromatography device and a single-pass crossflow diafiltration unit integrally integrated between the first and second chromatography device,
wherein the first chromatography device is configured to receive and process the first fluid and to provide a second fluid containing the target component,
the single-pass crossflow diafiltration unit is configured to continuously diafiltrate the second fluid with a diafiltration medium for obtaining a permeate and a third fluid containing the target component as a retentate; and
the second chromatography device is configured to receive and process the third fluid and to provide a fourth fluid containing the target component.

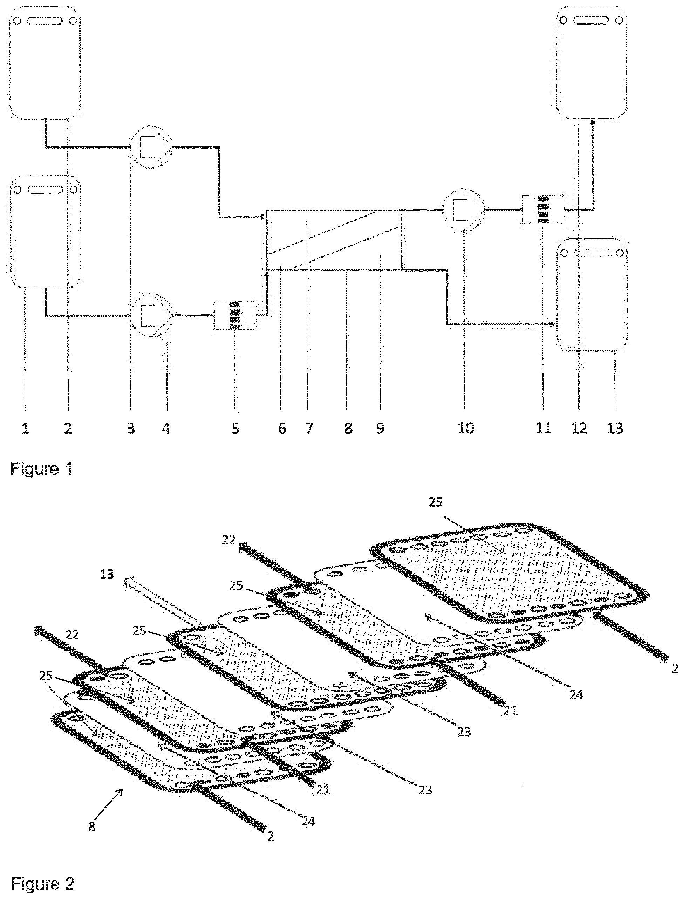

With the separation system of the present invention, it is preferably advantageously possible to directly combine two (different) chromatography devices in flow-through mode with an integrated buffer exchange between them by a single-pass tangential flow filtration (SPTFF) (cf. Figure 1). Due to this approach, three unit operations can be combined to one single unit operation. This preferably results in significant reductions in footprint and investment costs. Furthermore, preferably high yields of the target component can advantageously be achieved in a continuous flow-through operation mode. A first fluid containing the target component is subjected to purification by the first chromatography device to give a second fluid, which is immediately subjected to diafiltration in the crossflow diafiltration unit. The resulting third fluid is then subjected to purification by the second chromatography device to give a fourth fluid. The operations can advantageously be conducted in a continuous manner.

According to the present invention, the separation system comprises a first and a second chromatography device and a single-pass crossflow diafiltration unit integrally integrated between the first and second chromatography device. The term "integrally integrated" herein defines that the first and second chromatography device form one (closed) unit with the crossflow diafiltration unit. Accordingly, no further units or unit operations, such as in-line dilution, dilution in intermediate holding tanks, (additional) buffer exchange, titration, desalting steps, etc., are included between the first chromatography device and the crossflow diafiltration unit and between the crossflow diafiltration unit and the second chromatography device.

The first and the second chromatography device can each independently be a plurality of chromatography devices. One or more, preferably, one of the plurality of chromatography devices is/are operated at once. By applying switching means, it is possible to switch the chromatography devices within the respective plurality of chromatography devices, for example, in case one of the devices reached its capacity limit.

Any form and combination of chromatography methods and the respective ligands can be used for the first and the second chromatography device. For example, the first and the second chromatography method of the first and second chromatography device, respectively, can each independently be selected from the group consisting of anion exchange chromatography (AEX), cation exchange chromatography (CEX), hydrophobic interaction chromatography (HIC), affinity chromatography, mixed-mode chromatography (MMC), and size exclusion chromatography (SEC). Preferably, the first and the second chromatography method are selected from a combination of AEX and CEX, AEX and HIC, CEX and HIC, and each *vice versa.* More preferably, the first and the second chromatography method are selected from AEX and CEX, and CEX and AEX. With a combination of AEX and CEX and *vice versa* it is advantageously possible to remove impurities with different isoelectric points. With a combination of AEX and HIC and CEX and HIC it is advantageously possible to remove impurities/aggregates based on hydrophobicity. Examples of ligands for the above-mentioned methods are known in the art. For example, as ligand for strong AEX, quaternary ammonium can be used, and as ligand for strong CEX, sulfonic acid can be used.

The chromatography media of the first and the second chromatography device are not particularly limited. For example, the first and the second chromatography device can each independently comprise a chromatography medium selected from the group consisting of membrane adsorbers, chromatography resins, and monoliths. Preferably, the first and the second chromatography device apply the same chromatography medium. More preferably, the chromatography medium of each of the first and the second chromatography device is a membrane adsorber. Membrane adsorbers preferably allow significantly higher flow rates and a higher productivity. The first and the second chromatography device are preferably independently selected from the group consisting of AEX membrane absorber, CEX membrane absorber, HIC membrane absorber, affinity chromatography membrane absorber, MMC membrane absorber, and SEC membrane absorber, more preferably independently selected from AEX membrane absorber, CEX membrane absorber, and HIC membrane absorber, and even more preferably independently selected from AEX membrane absorber and CEX membrane absorber.

Preferably, the first chromatography device is configured to retain other components (impurity components) and to not substantially retain the target component to provide the second fluid containing the target component in a higher relative amount than in the first fluid. Herein, the term "relative amount" refers to the mass of the target component relative to the total mass of the other components (impurity components) in the respective fluid. Accordingly, the term "higher relative amount of the target component in the second fluid than in the first fluid" means that the mass of the target component relative to the total mass of the other components (impurity components) in the second fluid is higher than the mass of the target component relative to the total mass of the other components (impurity components) in the first fluid. This can for example be achieved by reducing the amount of the other components (impurity components) from the first fluid to the second fluid to a larger extend than the amount of the target component (if the amount of the target component is reduced at all).

In a preferred embodiment, the first chromatography device retains at least 20 mass%, more preferably at least 40 mass%, and most preferably at least 60 mass% of the other components (impurity components) present in the first fluid. Moreover, the first chromatography device retains at most 10 mass%, more preferably at most 5.0 mass%, more preferably at most 2.0 mass%, more preferably at most 1.0 mass%, and most preferably at most 0.10 mass% of the target component present in the first fluid.

Preferably, the second chromatography device is configured to retain other components (impurity components) and to not substantially retain the target component to provide the fourth fluid containing the target component in a higher relative amount than in the third fluid. The above definitions given with respect to the first and second fluid apply analogously.

In a preferred embodiment, the second chromatography device retains at least 20 mass%, more preferably at least 40 mass%, and most preferably at least 60 mass% of the other components (impurity components) present in the third fluid. Moreover, the second chromatography device retains at most 10 mass%, more preferably at most 5.0 mass%, more preferably at most 2.0 mass%, more preferably at most 1.0 mass%, and most preferably at most 0.10 mass% of the target component present in the third fluid.

The target component is not specifically limited. For example, the target component may be any biomolecule of interest, such as proteins, antibodies, hormones, vaccines, nucleic acids, exosomes, and viruses, and virus-like particles. In a preferred embodiment, the target component is an antibody, more preferably a monoclonal antibody (mAb), or fragments or derivatives thereof, or nanobodies. Examples of monoclonal antibodies are adalimumab, cetuximab, rituximab, infliximab, omalizumab, and denosumab. The target component can be obtained, for example, from cell cultures, such as "Chinese hamster ovary cells" (CHO cells) or other mammalian cell lines.

The source of the first fluid is not specifically limited. For example, the first fluid can be obtained by applying any biological, biochemical, chemical, or pharmaceutical method. Thereby, the first fluid can be obtained by previously conducting other purification methods applying different purification units. For example, the target component can be produced by appropriate cell lines, such as CHO cell lines e.g. by perfusion cultivation. As cell retention devices there can for example be applied an alternating flow filtration membrane. Moreover, as potential first capture steps, protein A affinity chromatography and virus inactivation by acidification (e.g. pH value < 3.5) can be performed. For obtaining any necessary (buffer) conditions in the first fluid, an optional diafiltration or titration can for example be performed.

The first fluid is not particularly limited as long as it contains a plurality of components, wherein at least one component of the plurality of components of the first fluid is the target component. The further (impurity) components (apart from the components of the medium) are not particularly limited and may depend on the preparation conditions of the target component. Examples of further components are aggregates, host cell proteins, desoxyribonucleic acid, as well as fragments and charge variants thereof.

The medium of the first fluid is not particularly limited. In principle, any fluid is suitable, with water and aqueous salt solutions being preferred. For example, an aqueous buffer solution can be used as the medium of the first fluid. Preferably, the medium of the first fluid is selected from the group consisting of KPI buffer, sodium phosphate buffer, sodium acetate buffer, PBS, glycine, citrate buffer, Tris buffer, BIS-Tris buffer, HEPES buffer, and water. The concentration of the buffer in the aqueous solution is not particularly limited and may for example be from 1.0 mM to 5.0 M, preferably from 5.0 mM to 1.0 M, more preferably from 10 mM to 200 mM, most preferably from 25 to 100 mM. The medium of the first fluid and the conditions thereof can be appropriately chosen depending on the components of the first fluid to be separated (impurity and target components) and the applied first chromatography device. For this purpose, there can for example be applied a statistical design of experiments (DoE) approach.

Design of Experiments (DoE) is an efficient method for determining the relevant influencing factors for a process or a product from a large number of parameters. With the aid of an experimental design, these factors are varied largely independently of one another in order to derive their effects on the target variables and, thus, result in a cause-effect model. During the evaluation, it is estimated whether all the intended targets can be achieved or whether, for example, certain targets are contradictory. Examples of targets may be high purity, high yield, high quality (e.g. if applicable: activity, critical quality attributes, glycosylation (e.g. for mAb), charge variants). Contradictory targets may be yield and purity. The aim of a DoE is to achieve the best trade-off between the targets. Commonly, a DoE consists of the following steps: screening of significant variables, analysis of the process model, optimization and validation.

The conditions of the first fluid can be appropriately set (e.g. by selecting an appropriate buffer, which may be evaluated by DoE) in order to optimize the yield and/or purity achieved by the first chromatography device.

The pH of the first fluid is not subject to any particular limitations. For example the pH of the first fluid may be from 3.0 to 14.0, preferably from 5.0 to 12.0, more preferably from 6.0 to 10.0, most preferably from 6.0 to 9.0. The pH of the second fluid may correspond to the pH of the first fluid, i.e. the pH may not be substantially altered by passing the first chromatography device.

The conductivity of the first fluid is not subject to any particular limitations and may be, for example, from 0 mS/cm to 500 mS/cm, preferably from 0 mS/cm to 150 mS/cm.

The crossflow diafiltration unit is a single-pass crossflow diafiltration unit and is configured to continuously diafiltrate the second fluid with a diafiltration medium for obtaining a permeate and a third fluid containing the target component as a retentate. Thus, the second fluid flows over the surface of the filter material and the third fluid is provided by the crossflow diafiltration unit after just a single pass, i.e. without recycling.

As single-pass crossflow diafiltration unit, there can be applied single-pass crossflow diafiltration units commonly known in the art. For example, there may be applied a crossflow diafiltration unit as described in WO 2017/174192 A1 in single-pass mode or as described in US 7 384 549 B2.

In a preferred embodiment, the single-pass crossflow diafiltration unit at least comprises a diafiltration channel, a (preferably flat) first filter material, a retentate channel, a (preferably flat) second filter material and a permeate collection channel, arranged in such a way that the first filter material delimits the diafiltration channel and the retentate channel from one another, and the second filter material delimits the retentate channel and the permeate collection channel from one another,
wherein the diafiltration channel is connected in a fluid conducting (communicating) manner to at least one inlet for the diafiltration medium; the retentate channel is connected in a fluid conducting manner to at least one inlet for the second fluid and to at least one outlet for the third fluid as the retentate; and the permeate collection channel is connected in a fluid conducting manner to at least one outlet for the permeate. An example of a respective crossflow diafiltration unit is for example schematically shown in Figure 2. Suitable crossflow diafiltration units are e.g. described in DE 10 2016 004 115 A1 (crossflow diafiltration unit for continuous diafiltration).

The shape (spatial form) of the first and second filter materials is not subject to any particular restriction. Preferably, the first filter material and the second filter material are flat filter materials. The term "flat" indicates that the respective filter material lies substantially in a single plane. Preferably, all filter materials lie more or less in planes that are substantially parallel to each other.

In addition, the crossflow diafiltration unit can be a wound module. For this purpose, an initially flat or planar arrangement of first and second filter material is wound around a core, so that first and second filter material (as well as diafiltration, retentate and permeate channels) each have a spiral cross-section.

In addition, the crossflow diafiltration unit may be a hollow fiber module. In this case, the first and second filter materials have a hollow fiber shape (cylinders without circular disk-shaped end sections). The inner diameters of the first and second hollow fiber filter materials are different to allow the first and second filter materials to be inserted into the second and first filter materials, respectively, to form the diafiltration, retentate, and permeate channels.

The first filter material has preferably a molecular weight cut-off (MWCO) in the range of 1 kDa to 1,500 kDa. The second filter material has preferably a molecular weight cutoff in the range of 1 kDa to 1,500 kDa. The determination of the molecular weight cut-off can be carried out in accordance with the US standard ASTM E1343-90 ("Standard test method for molecular weight cutoff evaluation of flat sheet ultrafiltration membranes"). The MWCO of the membranes is preferably selected such that the target component is retained in the retentate channel, while the smaller buffer components permeate through the membrane and are withdrawn in the permeate outlet.

The first filter material has preferably a pore size of 0.001 to 50 µm, preferably 0.01 to 0.5 µm. The second filter material has preferably a pore size of 0.001 to 50 µm, preferably 0.01 to 0.5 µm.

For pore sizes of at least 0.1 µm, i.e. for microfiltration membranes with an average pore size of 0.1 to 10 µm, capillary flux porometry is used to determine the pore size. This is a gas/liquid porosimetry in which the differential gas pressures and flow rates through a membrane sample are measured first in the wet and then in the dry state. Prior to measurement, the membrane sample is brought into contact with a wetting liquid in such a way that all pores are filled with this liquid. After the pores have been filled and the sample has been introduced, the measuring cell must be closed and the measurement started. After starting the measurement, the gas pressure is automatically and gradually increased and the pore diameters corresponding to the applied pressure are emptied by the gas pressure. This is continued until the relevant pore range has been covered, i.e. until even the smallest pores present in the measuring range have been freed of liquid. The pressure is then reduced again and the measurement is repeated automatically on the now dry sample. The pore size distribution is calculated from the difference between the two pressure-flow rate curves using the Young-Laplace equation (see also A. Shrestha, "Characterization of porous membranes via porometry", 2012, Mechanical Engineering Graduate Theses & Dissertations, Paper 38, University of Colorado at Boulder).

For the determination of pore sizes larger than 10 µm and up to 1 mm, the image analysis based method described in Journal of Membrane Science 372 (2011), pages 66 to 74, can be used.

For pore sizes less than 0.1 µm, the liquid-liquid displacement method, which has similarities to capillary flow porometry, is used. However, instead of the gas flow rates, the flow rates of the displacing liquid are measured as a function of the differential pressure increase (see also R. Davila, "Characterization of ultra and nanofiltration commercial filters by liquid-liquid displacement porosimetry", 2013).

According to a preferred embodiment, the first filter material is a first filtration membrane or the second filter material is a second filtration membrane. It is particularly preferred that the first filter material is a first filtration membrane and that the second filter material is a second filtration membrane.

In particular, a porous membrane in the ultrafiltration and microfiltration range is suitable as the first filter material and as the second filter material.

In a preferred embodiment, the second filter material has a pore size being smaller than the target component and being larger than at least one of the other components of the second fluid. Preferably, the first and the second filter material are identical.

The ultrafiltration membranes are characterized by pore sizes of less than 0.01 µm or by molecular weight cut-offs that are approximately in the molecular weight range of 1 to 1,500 kDa, while the microfiltration membranes have pore sizes in the range of 0.01 to 50 µm, preferably 0.01 to 0.5 µm, or molecular weight cut-offs of 30 to 1,500 kDa. The filtration membranes may consist, for example, of polyvinylidene fluoride, cellulose and derivatives thereof, polyethersulfone or polysulfone, with crosslinked cellulose hydrate being particularly preferred.

The inlet for the feed fluid (here: second fluid) is mounted preferably in a first edge region of the crossflow diafiltration unit; and the outlet for the retentate (here: third fluid) is mounted in a second edge region of the crossflow diafiltration unit that faces the first edge region. Owing to this arrangement it is possible to define a substantially uniform direction of flow of the retentate from the inlet for the feed fluid, as the starting point, to the outlet of the retentate, as an end point. As a result, the direction of flow of the retentate runs substantially parallel to the flow path along the (flat) filter material, i.e., in essence without deflections, so that a stable and reliable flow of the retentate can be ensured by the crossflow diafiltration unit. In addition, due to the substantially linear flow path without deflections, loops or the like, it is possible to minimize the pressure drop in the filtration unit as well as undesired effects of non-linear flows on the target substances, contained in the feed fluid. For the above reasons it is also preferred that the inlet for the diafiltration medium be mounted in the first edge region of the crossflow diafiltration unit. However, it is also possible to mount the inlet for the diafiltration medium in the second edge region or in the third and/or fourth edge region.

According to a preferred embodiment, the outlet for the permeate is mounted in the second edge region of the crossflow diafiltration unit. It is particularly preferred that in each case at least one outlet for the permeate be mounted in both the first and the second edge region of the crossflow diafiltration unit. In another embodiment of the invention the outlets for the permeate are mounted, as an alternative or in addition, in the third and/or fourth edge region of the crossflow diafiltration unit. The third edge region is located on the left side of the flow direction in a plan view of the crossflow diafiltration unit from the side of the diafiltration channel. Correspondingly the fourth edge region is located on the right side and, thus, lies opposite the third edge region. The above arrangement of the outlet(s) makes it possible to achieve not only a particularly high permeate performance, but also design advantages.

The first edge region comprises preferably the outer third of the length of the filtration unit opposite to the direction of flow. Correspondingly the second edge region comprises the outer third of the length of the filtration unit along the direction of flow. The same applies to the third and fourth edge regions. It is advantageous to make the first to fourth edge regions as small as possible. Therefore, it is particularly preferred that the edge regions comprise the respective outer 20%, even more preferably the respective outer 10%, and most preferably the respective outer 3%.

In principle, there is no particular constraint on the mounting of the inlets and outlets. For example, the inlets and outlets may be mounted in such a way that the feed fluid already enters the retentate channel in the direction of flow and leaves it in the direction of flow. Correspondingly, the outlet for the permeate can be mounted in such a way that the permeate leaves the permeate collection channel in the direction of flow; and/or the inlet for the diafiltration medium can be mounted in such a way that it enters the diafiltration channel in the direction of flow. Preferably, however, the inlets and outlets are mounted in such a way that the diafiltration medium enters the diafiltration channel perpendicular to the direction of flow; and then the feed fluid enters the retentate channel perpendicular to the direction of flow and leaves it, as a retentate, perpendicular to the direction of flow. Such a mounting of the inlets and outlets facilitates the arrangement of a plurality of the inventive filtration units to form a filter cassette. A respective arrangement is e.g. schematically shown in Figure 2. As shown in Fig. 2, the second fluid is supplied via the feed inlet. Due to the selected MWCO of the membrane, the target component is retained in the retentate channel, while smaller components of the second fluid permeate through the membrane and are withdrawn in the permeate outlet. Thereby, the purity of the target component is advantageously increased. Simultaneously, the diafiltration buffer is actively supplied by an additional channel, permeates through the membrane and washes out other components of the second fluid (including buffer components) from the retentate to the permeate (buffer exchange).

Preferably the crossflow diafiltration unit comprises a plurality of inlets for the feed fluid (here: second fluid), a plurality of outlets for the retentate (here: third fluid), a plurality of inlets for the diafiltration medium, and a plurality of outlets for the permeate in each of the retentate channel, the diafiltration channel, and the permeate channel, respectively. The respective plurality of inlets is supplied with fluid/medium by one respective inlet channel which branches into the respective plurality of inlets. The respective plurality of outlets combines to one respective outlet channel and supplies the respective fluid thereto.

In continuous diafiltration both the feed fluid (here: second fluid) and the diafiltration medium are added continuously so that the process does not have to be interrupted. As a result, the crossflow diafiltration unit makes it possible to run the process in an efficient and economical manner.

The diafiltration medium is not particularly limited and is chosen in accordance with the second chromatography device. In principle, any fluid is suitable, with water and aqueous salt solutions being preferred. For example, an aqueous buffer solution can be used as the diafiltration medium. Preferably, the diafiltration medium is selected from the group consisting of KPI buffer, sodium phosphate buffer, sodium acetate buffer, PBS, glycine, citrate buffer, Tris buffer, BIS-Tris buffer, HEPES buffer, and water. The concentration of the buffer in the aqueous solution is not particularly limited and may for example be from 1.0 mM to 5.0 M, preferably from 5.0 mM to 1.0 M, more preferably from 10 mM to 200 mM, most preferably from 25 to 100 mM. The diafiltration medium and the conditions thereof can be appropriately chosen depending on the components of the third fluid to be separated (impurity and target components) and the applied second chromatography device. For this purpose, there can for example be applied a statistical design of experiments (DoE) approach as mentioned above.

The conditions of the diafiltration medium and of the third fluid can be appropriately set (e.g. by selecting an appropriate buffer, which may be evaluated by DoE) in order to optimize the yield and/or purity achieved by the second chromatography device.

The pH of the diafiltration medium and the third fluid is not particularly limited. For example, pH of the diafiltration medium may be from 3.0 to 14.0, preferably from 5.0 to 12.0, more preferably from 6.0 to 10.0, most preferably from 6.0 to 9.0. The pH of the third fluid may be from 3.0 to 14.0, preferably from 5.0 to 12.0, more preferably from 6.0 to 10.0, most preferably from 6.0 to 9.0.

The conductivity of the diafiltration medium and the third fluid is not subject to any particular limitations and may be, for example, from 0 mS/cm to 500 mS/cm, preferably from 0 mS/cm to 150 mS/cm.

Preferably, the second and third fluid differ in at least one property selected from the group consisting of pH, conductivity, salt concentration (e.g. ammonium sulfate), and buffer composition.

For example, in case the first and second chromatography devices are a combination of AEX and CEX or of CEX and AEX, the pH can, in particular, be changed from the second fluid to the third fluid. This advantageously preferably gives the opportunity to remove impurities with different isoelectric points. In case the first and second chromatography devices are a combination of AEX and CEX or of CEX and AEX, the conductivity can, in particular, be changed from the second fluid to the third fluid. This advantageously preferably gives the opportunity to remove impurities based on the affinity to the ligand (ionic interaction). In case the first and second chromatography devices are a combination of AEX and HIC or of CEX and HIC, the ammonium sulfate concentration can, in particular, be changed from the second fluid to the third fluid. This advantageously preferably gives the opportunity to remove impurities/aggregates based on hydrophobicity. In case the first and second chromatography devices are a combination of AEX and HIC or of CEX and HIC, the conductivity can, in particular, be changed from the second fluid to the third fluid. This advantageously preferably gives the opportunity to remove impurities/aggregates based on hydrophobicity. In case the first and second chromatography devices are a combination of HIC and AEX or of HIC and CEX, ammonium sulfate can, in particular, be removed or the conductivity can, in particular, be lowered from the second fluid to the third fluid. This advantageously preferably gives the opportunity to bind the impurities on AEX/CEX ligands (impurity removal).

In a preferred embodiment, the crossflow diafiltration unit is configured such that a volume flow rate of the supplied diafiltration medium is 0.5 to 20 times a volume flow rate of the supplied second fluid (i.e. the supplied feed fluid). The volume flow rate of the supplied diafiltration medium is preferably 1.0 to 15, more preferably 2.0 to 10, more preferably 4.0 to 9.0, most preferably 5.0 to 7.0 times the volume flow rate of the supplied second fluid.

The volume flow rates are not limited to any particular value and may depend on the exploit size of the chromatography devices and diafiltration units. For example, the volume flow rate of the supplied diafiltration medium can be from 1.0 mL/min to 2.0 L/min, preferably from 2.0 mL/min to 1.0 L/min, more preferably from 4.0 mL/min to 500 mL/min, most preferably from 10 mL/min to 175 mL/min. For example, the volume flow rate of the supplied second fluid can be from 0.5 mL/min to 100 mL/min, preferably from 1.0 mL/min to 50 mL/min, more preferably from 2.0 mL/min to 25 mL/min. For example, the volume flow rate of the retentate/third fluid at the outlet can be from 0.5 mL/min to 100 mL/min, preferably from 1.0 mL/min to 50 mL/min, more preferably from 2.0 mL/min to 25 mL/min.

In a preferred embodiment, the diafiltration medium is supplied at a pressure of 0.1 to 4 bar. More preferably, the diafiltration medium is supplied at a pressure that is greater than the retentate/third fluid outlet pressure.

Preferably, diafiltration is carried out continuously, i.e., under constant/continuous addition of the diafiltration medium and the feed fluid, so that a particularly efficient and economical filtration method can be provided.

After crossflow diafiltration, the (majority of the) target component is preferably contained in the retentate (third fluid). Thus, the mass ratio of the mass of the target component included in the retentate (third fluid) to the total mass of the target component subjected to diafiltration (i.e. in the second fluid) is preferably more than 50 mass%, more preferably at least 80 mass%, more preferably at least 90 mass%, most preferably at least 95 mass%.

The means for providing the fluids and media are not particularly limited. For example, as means for providing (including passing to) the fluids and media, there can be used pressure tanks, pumps, and valves. Valves can be used instead of e.g. retentate pumps and can control the pressure. In a preferred embodiment, the system comprises at least three means, more preferably three means, for providing the fluids and media. In particular, for providing the third fluid to the second chromatography medium, there can be used a valve or a pump. Preferably, pumps are used for providing the fluids and media. In a preferred embodiment, the system comprises at least three pumps, more preferably three pumps. For example, there is provided a pump for each of the first fluid, the diafiltration medium, and the third fluid. A respective example is for example shown in Figure 1. This system can be operated with any kind of pumps as well as stand-alone pumps or more complex systems that have at least three pumps. By modifying the flow rates of the three pumps, the desired buffer exchange rate as well as product concentration or dilution can be adjusted. At each position for the means for providing the fluids and media, there can be provided means for monitoring the conditions of the respective fluid/media, such as pH meters, conductivity sensors, pressure sensors, spectrometers (e.g. Raman, UV/VIS, NIR), and flow meters.

The fourth fluid can be subjected to further purification steps, such as affinity and/or hydrophobic interaction and/or cation and/or anion exchange chromatography and/or sterile filtration and/or virus filtration and/or virus inactivation and/or precipitation and/or crystallization and/or extraction (aqueous two-phase extraction) and/or lyophilization in any suitable order. Accordingly, the separation system of the present invention can further comprise respective further purification units, such as affinity and/or hydrophobic interaction and/or cation and/or anion exchange chromatography units and/or sterile filtration and/or virus filtration units and/or virus inactivation units and/or precipitation units and/or crystallization units and/or extraction units (aqueous two-phase extraction units) and/or lyophilization units in any suitable order. In-between the respective units further single-pass crossflow diafiltration units can be included.

Apart from the first and second chromatography devices and the single-pass crossflow diafiltration unit, the separation system can include further such chromatography devices and single-pass crossflow diafiltration units. For example, the fourth fluid obtained from the second chromatography device can be provided to a further single-pass crossflow diafiltration unit, wherein the fourth fluid is continuously diafiltrated for obtaining a second permeate and a fifth fluid containing the target component. The fifth fluid can then be provided to a third chromatography device, wherein it is processed to provide a sixth fluid containing the target component. This can even be further extended in the above-described manner.

In a further aspect, the present invention relates to a method for purifying a target component included in a first fluid, wherein the method comprises the steps of
(a) providing the first fluid to a first chromatography device,
(b) processing the first fluid in the first chromatography device to obtain a second fluid containing the target component,
(c) providing the second fluid to a single-pass crossflow diafiltration unit,
(d) processing the second fluid with a diafiltration medium in the single-pass crossflow diafiltration unit to obtain a third fluid containing the target component,
(e) providing the third fluid to a second chromatography device,
(f) processing the third fluid in the second chromatography device to obtain a fourth fluid containing the target component,
wherein the single-pass crossflow diafiltration unit is integrally integrated between the first and second chromatography device. The above statements and definitions analogously apply to this aspect of the present invention. Preferably, the method according to the present invention is carried out by using the separation system according to the present invention.

The method of the present invention can further comprise steps of carrying out DoE with respect to the target component. For example, these steps can comprise the following steps: screening of significant variables, analysis of the process model, optimization and validation. As a result, there can preferably be obtained, for a specific target component, the optimized (e.g. with respect to yield and/or purity) first and second chromatography devices, other components of the fluids, and/or diafiltration medium.

In a further aspect, the present invention relates to the use of a single-pass crossflow diafiltration unit for integrally connecting a first and a second chromatography device suitable for purifying a target component contained in a first fluid, wherein the first chromatography device is configured to receive and process the first fluid and to provide
a second fluid containing the target component,
the crossflow diafiltration unit is configured to continuously diafiltrate the second fluid with a diafiltration medium for obtaining a permeate and a third fluid containing the target component as a retentate; and
the second chromatography device is configured to receive and process the third fluid and to provide a fourth fluid containing the target component. The above statements and definitions analogously apply to this aspect of the present invention. Preferably, the single-pass crossflow diafiltration unit is the single-pass crossflow diafiltration unit of the separation system according to the present invention. Preferably, the first and the second chromatography device are the first and the second chromatography device of the separation system according to the present invention.

### Reference signs

### Figures 1 and 2

1 = First fluid (buffer solution for first chromatography device)
2 = Diafiltration medium (second buffer solution)
3 = Pump for diafiltration medium
4 = Pump for first fluid
5 = First chromatography device with first chromatography medium
6 = Retentate channel
7 = Diafiltration channel
8 = Single-pass tangential flow filtration cassette
9 = Permeate channel
10 = Pump for third fluid (retentate)
11 = Second chromatography device with second chromatography medium
12 = Fourth fluid (flow-through of the second chromatography device (containing the purified target component))
13 = Permeate
21 = Second fluid
22 = Retentate /third fluid
23 = Second filter material
24 = First filter material
25 = Spacers

Fig. 1: Schematic representation of the separation system of the present invention. A direct combination of two different chromatography media in flow-through mode with an integrated buffer exchange between them by a single path tangential flow filtration.
Fig. 2: A possible structure of a single-pass crossflow diafiltration unit (8) of the present invention with a flat second filter material (23) and a flat first filter material (24), wherein the streams of diafiltration medium (2), second fluid (21), retentate/third fluid (22) and permeate (13) are illustrated by arrows. The diafiltration channel, the retentate channel and the permeate collection channel are kept open by spacers (25) for the respective media.

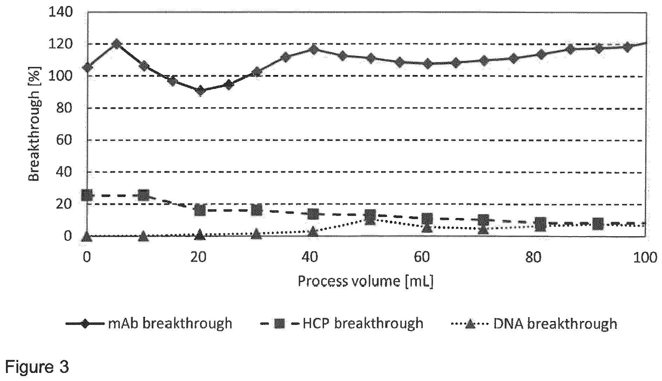

Fig. 3: Breakthrough curves for the mAb as product and DNA as well as HCP as impurities after the second chromatography step of the Inventive Example. Breakthrough was determined by the ratio of the concentration from the flow-through of the second chromatography device to the concentration of the feed solution.

The present invention will be further illustrated in the following example without being limited thereto.

### Inventive Example

A CHO cell line was used to produce a mAb in perfusion cultivation. Cells were cultivated using a commercial serum-free medium at 36.8°C and pH 6.95. An alternating flow filtration membrane was used as cell retention device to collect the perfusion permeate with the mAb as product. As first capture step, a protein A affinity chromatography and subsequently a virus inactivation by acidification (pH 3.4) was performed. Afterwards a diafiltration was performed to obtain the mAb in the appropriate buffer conditions for the first chromatography medium. Thereby, the first fluid was obtained.

As first chromatography medium an AEX membrane adsorber (Sartobind Q; strong anion exchanger, ligand: quaternary ammonium, membrane material: stabilized strengthened cellulose, pore size: 3-5 µm, ligand density 2-5 µeq/cm²) and as second chromatography medium an CEX membrane adsorber (Sartobind S; strong cation exchanger, ligand: sulfonic acid, membrane material: stabilized strengthened cellulose, pore size: 3-5 µm, ligand density 2-5 µeq/cm²) were used. The appropriate buffer conditions were obtained by a DoE based approach. In this context, a conductivity of 4.2 mS/cm at a pH of 8.0 was evaluated for the first chromatography medium and a conductivity of 4.0 mS/cm at a pH of 8.5 was evaluated for the second chromatography medium.

The setup of the process is shown in Figure 1. Both chromatography steps and the buffer exchange in-between were performed in just one unit operation instead of three (first chromatography step, diafiltration for buffer exchange and second chromatography step) by the use of a system comprising 3 pumps. Throughout the complete process, in the flow-through of the second chromatography device, a high yield of the mAb was obtained. This was indicated by the high breakthrough of the mAb shown in Figure 3 (Fluctuations and values slightly above 100 % were due to slight fluctuations of the pump flowrate). Simultaneously, throughout the process, a high removal of HCP (88 %, corresponding to an average amount of 29 ppm in the second fluid over all fractions) and of DNA (96 %, corresponding to an average amount of 2 ppm in the fourth fluid over all fractions) was obtained.

## Claims

1. A separation system configured to process a first fluid (1) containing a plurality of components, wherein at least one component of the plurality of components of the first fluid (1) is a target component and wherein the separation system comprises:
a first (5) and a second (11) chromatography device and a single-pass crossflow diafiltration unit (8) integrally integrated between the first (5) and second (11) chromatography device,
wherein the first chromatography device (5) is configured to receive and process the first fluid (1) and to provide a second fluid (21) containing the target compound,
the single-pass crossflow diafiltration unit (8) is configured to continuously diafiltrate the second fluid (21) with a diafiltration medium (2) for obtaining a permeate (13) and a third fluid (22) containing the target component as a retentate; and
the second chromatography device (11) is configured to receive and process the third fluid (22) and to provide a fourth fluid (12) containing the target compound.

2. A method for purifying a target component included in a first fluid (1), wherein the method comprises the steps of
(a) providing the first fluid (1) to a first chromatography device (5),
(b) processing the first fluid (1) in the first chromatography device (5) to obtain a second fluid (21) containing the target component,
(c) providing the second fluid (21) to a single-pass crossflow diafiltration unit (8),
(d) processing the second fluid (21) with a diafiltration medium (2) in the single-pass crossflow diafiltration unit (8) to obtain a third fluid (22) containing the target component,
(e) providing the third fluid (22) to a second chromatography device (11),
(f) processing the third fluid (22) in the second chromatography device (11) to obtain a fourth fluid (12) containing the target component,
wherein the single-pass crossflow diafiltration unit (8) is integrally integrated between the first (5) and second (11) chromatography device.

3. Use of a single-pass crossflow diafiltration unit (8) for integrally connecting a first (5) and a second (11) chromatography device suitable for purifying a target component contained in a first fluid (1), wherein the first chromatography device (5) is configured to receive and process the first fluid (1) and to provide a second fluid (21) containing the target component,
the single-pass crossflow diafiltration unit (8) is configured to continuously diafiltrate the second fluid (21) with a diafiltration medium (2) for obtaining a permeate (13) and a third fluid (22) containing the target component as a retentate; and
the second chromatography device (11) is configured to receive and process the third fluid (22) and to provide a fourth fluid (12) containing the target component.

4. The separation system according to claim 1, or the method according to claim 2, or the use according to claim 3, wherein the single-pass crossflow diafiltration unit at least comprises a diafiltration channel (7), a first filter material (24), a retentate channel (6), a second filter material (23) and a permeate collection channel (9), arranged in such a way that the first filter material (24) delimits the diafiltration channel (7) and the retentate channel (6) from one another, and the second filter material (23) delimits the retentate channel (6) and the permeate collection channel (9) from one another,
wherein the diafiltration channel (7) is connected in a fluid conducting manner to at least one inlet for the diafiltration medium (2); the retentate channel (6) is connected in a fluid conducting manner to at least one inlet for the second fluid (21) and to at least one outlet for the third fluid (22); and the permeate collection channel (9) is connected in a fluid conducting manner to at least one outlet for the permeate (13).

5. The separation system according to claim 4, or the method according to claim 4, or the use according to claim 4, wherein the second filter material (23) has a pore diameter being smaller than the target component and being larger than at least one of the other components of the second fluid (21).

6. The separation system according to any of claims 1, 4, and 5, or the method according to any of claims 2, 4, and 5, or the use according to any of claims 3 to 5, wherein the second (21) and third (22) fluid differ in at least one property selected from the group consisting of pH, conductivity, salt concentration, and buffer composition.

7. The separation system according to any of claims 1 and 4 to 6, or the method according to any of claims 2 and 4 to 6, or the use according to any of claims 3 to 6, wherein the first (5) and the second (11) chromatography device are each independently a plurality of chromatography devices.

8. The separation system according to any of claims 1 and 4 to 7, or the method according to any of claims 2 and 4 to 7, or the use according to any of claims 3 to 7, wherein, in each of the first (5) and the second (11) chromatography device, a chromatography method is independently selected from the group consisting of anion exchange chromatography, cation exchange chromatography, hydrophobic interaction chromatography, affinity chromatography, mixed-mode chromatography, and size exclusion chromatography.

9. The separation system according to claim 8, or the method according to claim 8, or the use according to claim 8, wherein, in the first (5) and the second (11) chromatography device, the chromatography method is selected from AEX and CEX, and CEX and AEX.

10. The separation system according to any of claims 1 and 4 to 9, or the method according to any of claims 2 and 4 to 9, or the use according to any of claims 3 to 9, wherein the first (5) and the second (11) chromatography device each independently comprise a chromatography medium selected from the group consisting of membrane adsorbers, chromatography resins, and monoliths.

11. The separation system according to claim 10, or the method according to claim 10, or the use according to claim 10, wherein the first and the second chromatography device are independently selected from AEX membrane absorber and CEX membrane absorber.

12. The separation system according to any of claims 1 and 4 to 11, or the method according to any of claims 2 and 4 to 11, or the use according to any of claims 3 to 11, wherein the system comprises at least three means for providing the fluids and media selected from the group consisting of pressure tanks, pumps, and valves.

13. The separation system according to claim 12, or the method according to claim 12, or the use according to claim 12, wherein three pumps (3, 4, 10) are used as the means for providing the fluids and media.

14. The separation system according to any of claims 1 and 4 to 13, or the method according to any of claims 2 and 4 to 13, or the use according to any of claims 3 to 13, wherein the single-pass crossflow diafiltration unit is configured such that a volume flow rate of the supplied diafiltration medium is 0.5 to 20 times a volume flow rate of the supplied second fluid.

15. The separation system according to any of claims 1 and 4 to 14, or the method according to any of claims 2 and 4 to 14, or the use according to any of claims 3 to 14, wherein the target component is selected from proteins, antibodies, hormones, vaccines, nucleic acids, exosomes, viruses, and virus-like particles.
